# EUROPÄISCHE PATENTSCHRIFT

(11) **EP 0 806 953 B1**
(45) Veröffentlichungstag und Bekanntmachung des Hinweises auf die Patenterteilung: **27.06.2001**
(21) Anmeldenummer: 95943170.1
(22) Anmeldetag: 20.12.1995
(51) Int. Cl.: A61K 31/59

(54) **MITTEL ZUR BEEINFLUSSUNG VON STÖRUNGEN DER KNOCHENBILDUNG, DAS KALIUMCITRATE UND/ODER KALIUMLACTATE UND/ODER KALIUMMALATE ENTHÄLT**
AGENT FOR ACTING UPON BONE FORMATION DISTURBANCES CONTAINING POTASSIUM CITRATES, LACTATES AND/OR MALATES
SUBSTANCE PERMETTANT D'AGIR SUR LES TROUBLES DE L'OSSIFICATION, CONTENANT DES CITRATES, DES LACTATES ET/OU DES MALATES DE POTASSIUM

(30) Priorität: 01.02.1995 DE 19503190
(43) Veröffentlichungstag der Anmeldung: 19.11.1997
(73) Patentinhaber: Orthomol Pharmazeutische Vertriebs Gmbh, 40764 Langenfeld (DE)
(72) Erfinder: DIETL, Hans, D-83043 Bad Aibling (DE)
(74) Vertreter: Weisert, Annekäte, Dipl.-Ing. Dr.-Ing.
(86) Internationale Anmeldenummer: EP9505049
(87) Internationale Veröffentlichungsnummer: WO9623504

(56) Entgegenhaltungen:
- EP-A- 0 335 140
- EP-A- 0 390 930
- EP-A- 0 507 157
- EP-A- 0 704 199
- WO-A-92/21355
- US-A- 5 389 391
- CLINICAL AND EXPERIMENTAL RHEUMATOLOGY, Bd. 9, Nr. 6, 1991, Seiten 625-627, XP000577539 MONTE NETO ET AL: "OSTEOMALACIA SECONDARY TO RTA IN A PATIENT WITH PRIMARY SJÖGREN'S SYNDROME"
- DATABASE WPI Week 9338 Derwent Publications Ltd., London, GB; AN 93-299554 XP002009375 & JP,A,05 213 763 (SANWA KAGAKU KENKYUSHO CO) , 24.August 1993
- DATABASE WPI Week 7712 Derwent Publications Ltd., London, GB; AN 77-21064 XP002009376 & JP,A,52 018 811 (EISAI KK) , 12.Februar 1977

## Beschreibung

Die Erfindung betrifft ein Mittel zur Prophylaxe und/oder Therapie von Zuständen, die mit einer Störung der Knochenbildung einhergehen.

Störungen des Knochenstoffwechsels, insbesondere in Form des Auftretens einer Osteoporose, sind in den westlichen Industrieländern weit verbreitet. Osteoporose ist eine Erkrankung, die gekennzeichnet ist durch Störungen im Knochenaufbau sowie durch einen gesteigerten Knochenabbau. Bei einer Störung des Knochenaufbaus kommt es beispielsweise zu einer verminderten Synthese der Proteine des Knochen, z.B. Kollagen, Elastin, Osteocalcin sowie des Einbaus von Calcium und Phosphat. Eine solche Synthese- und/oder Einbauminderung führt zu einer oder verstärkt eine Osteoporose. Wichtig für die Proteinsynthes und den Einbau von Calcium und Phosphat sind beispielsweise die Vitamine D und K. Beim Knochenabbau wird neben Calcium auch Phosphat vermehrt ausgeschieden, zudem nehmen die Knochenproteine ab. Die Osteoporose stellt somit ein multifaktorielles Geschehen dar, wobei der Abbau von Knochenmasse und die Verringerung der Knochendichte im Vordergrund stehen. Die jährliche Abbaurate beträgt dabei 1 % bis 2 %; sie kann jedoch auf ein Vielfaches davon ansteigen und wird im Hinblick auf eine Osteoporose dramatisch, wenn sie 3 % bis 4 % übersteigt. Beim Knochenabbau wird vor allem Calcium aus dem Knochen herausgelöst, der dadurch leicht brüchig werden kann.

Eine weitere Erkrankung mit einer gestörten Knochenbildung ist die Osteomalazie, die durch einen mangelhaften Einbau von Mineralstoffen in das normal oder überschießend gebildete Eiweißknochengrundgerüst gekennzeichnet ist. Dadurch kommt es zu breiten unverkalkten Osteoidsäumen und unter allgemeiner Zunahme der elastischen Osteoidsubstanz zu erhöhter Weichheit und Verbiegungstendenz der Knochen.

Gewöhnlich tritt eine Osteoporose im sechsten Lebensjahrzehnt auf; bei Männern tritt sie wegen der größeren Knochenmasse bzw. Knochendichte und der andauernden Testosteronproduktion später und weit weniger heftig auf als bei Frauen.

Neben der Gabe von östrogenen bei postmenopausalen Frauen sowie hochdosierten Fluoriden, werden bei der Prophylaxe und Therapie von Knochenstoffwechselstörungen und des Knochenabbaus vor allem Calcium und Vitamin D, häufig in Kombination, verwendet.

So wird eine Erhöhung der täglichen Calciumaufnahme mit der Nahrung von ca. 600 bis 800 mg auf ca. 1000 bis 1500 mg empfohlen, wobei diese Zufuhrmengen durch einen erhöhten Verzehr von Milch und Milchprodukten erreicht werden können. Da Milch auch Vitamin D enthält, steigt damit auch die Zufuhr von Vitamin D.

Da viele Personen jedoch Milch und Milchprodukte nicht in den ausreichenden Mengen zu sich nehmen, werden statt dessen häufig auch Calciumsalze und Vitamin D in isolierter Form zusätzlich als Nahrungsergänzung bzw. als Arzneimittel eingenommen. Dadurch wird die Knochenabbaurate vermindert. Trotzdem schreitet der Abbau, wenn auch in geringerem Maße fort. Es ist daher wünschenswert, neben der Gabe von Calcium und Vitamin D weitere möglichst gut verträgliche Substanzen zu verabreichen, welche den Knochenabbau, charakterisiert durch den Verlust von Calcium aus dem Knochen, zusätzlich verringern.

Der Erfindung liegt daher die Aufgabe zugrunde, ein Mittel zur Prophylaxe und/oder Therapie von Zuständen, die mit einer Störung der Knochenbildung einhergehen, insbesondere zur Beeinflussung des Knochenabbaus, bereitzustellen. Das Mittel soll ferner keine Nebenwirkungen besitzen und sich auch für die Langzeitverabreichung eignen und für den Patienten bequem und einfach einzunehmen sein. Außerdem soll das Mittel auch mit anderen vom Patienten gegebenenfalls eingenommenen Arzneimitteln verträglich sein. Ferner soll sich das erfindungsgemäße Mittel für eine Verwendung als Nahrungsergänzung oder diätetisches Lebensmittel eignen.

Überraschenderweise wurde nun gefunden, daß sich durch die Aufnahme von Kaliumcitraten und/oder Kaliumlactaten und/oder Kaliummalaten, insbesondere von Kaliumcitraten und/oder Kaliumlactaten, der

Verlust von Calcium aus dem Körper und damit der Knochenabbau vermindern läßt. Ferner wurde überraschenderweise festgestellt, daß auch bereits die niedrig dosierte Gabe von Vitamin K (Phyllochinon) eine Wirkung auf den Caiciumstoffwechsel dahingehend besitzt, daß dadurch der Knochenabbau verringert und der Wiederaufbau des Knochens begünstigt wird.
Gegenstand der Erfindung ist daher ein Mittel zur Prophylaxe und/oder Therapie von Zuständen, die mit einer Störung der Knochenbildung einhergehen, enthaltend
i) Kaliumcitrat, -lactat und/oder -malat,
ii) Vitamin D und
iii) ein Calciumsalz. Ferner betrifft die Erfindung die Verwendung von Kaliumcitraten und/oder Kaliumlactaten und/oder Kaliummalaten zusammen mit Calciumsalzen und Vitamin D zur Prophylaxe und/oder Therapie von Erkrankungen, die mit einer Störung der Knochenbildung einhergehen sowie des Knochenabbaus. Bevorzugt betrifft die Erfindung ein Mittel zur Prophylaxe und/oder Therapie von Osteoporose oder Osteomalazie.

Die genannten Kaliumcitrate sind gut verträglich; dies ist deshalb von ganz besonderer Bedeutung, da es sich beim Knochenabbau wie der Osteoporose um einen sich langsam und langfristig entwickelnden Vorgang handelt, der nur durch die Langzeitanwendung entsprechender Substanzen zu beeinflussen ist.

Die Kaliumsalze haben keine Nebenwirkungen; sie sind als Lebensmittelzusatzstoffe zugelassen und können daher sogar prophylaktisch in diätetischen Lebensmitteln oder als Nahrungsergänzung eingesetzt werden. Zudem lassen sie sich auch im Hinblick auf den Geschmack gut verarbeiten. Dies ist besonders im Hinblick auf die notwendige langandauernde Verabreichung wichtig.

Die Kaliumcitrate bzw. -lactate bzw. -malate verringern die Ausscheidung von Calcium im Urin, verbessern dadurch die Calciumbilanz und verringern die Abbaurate der Knochen, da aus dem Knochen weniger Calcium freigesetzt wird. Zudem erhöhen sie Osteocalcin, ein Protein, welches den Aufbau des Knochens fördert und damit der Entstehung und dem Fortschreiten einer Osteoporose entgegenwirkt.

Kaliumcitrate und -lactate sind bevorzugt, da in manchen Fällen (z. B. bei gleichzeitigem Bluthochdruck) die Zufuhr von Natrium in höheren Mengen unerwünscht ist.

Die Dosierung der Kaliumcitrate und/oder -lactate und/oder -malate beträgt üblicherweise 10 mmol bis 100 mmol/Tag, berechnet als mmol Kalium. Bevorzugt sind 20 bis 50 mmol/Tag.

Als Kaliumcitrate können Mono-, Di- und Tricitrate des Kaliums verwendet werden, bevorzugt sind die Tricitrate, z.B. Trikaliumcitrate.

Die Kaliumsalze sollen im Gemisch mit anderen knochenabbauvermindernden Substanzen wie Calciumsalzen und/oder Vitamin D eingenommen werden.

Es ist bekannt, daß Vitamin K über die Bildung des Proteins Osteocalcin bei der Regelung der Knochenmineralisation eine Rolle spielt. Bisher wurde jedoch angenommen, daß ein Mangel an Vitamin K praktisch nie auftritt und die zusätzliche Verabreichung von Vitamin K den Knochenstoffwechsel daher nicht beeinflußt.

Überraschenderweise wurde nun erfindungsgemäß gefunden, daß bereits die Verabreichung geringer Mengen an Vitamin K, nämlich nur 50 bis 300 *µ*g/Tag, den Knochenabbau vermindert. Dabei können alle Formen des Vitamin K eingesetzt werden. Am besten wird Vitamin K in Form des gut verträglichen Vitamins K₁ (Phyllochinon) eingesetzt. Dabei ist es besonders günstig, das Vitamin K₁ zusammen mit den Kaliumsalzen, Calciumsalzen und Vitamin D einzusetzen, um eine optimale Wirkung zu erzielen. Hierbei können sämtliche Formen des Vitamin D eingesetzt werden. Bevorzugt wird jedoch Vitamin D₃ (Cholecalciferol) verabreicht.

Das erfindungsgemäße Mittel wird auf oralem Weg verabreicht und kann auf jede für diesen Verabreichungsweg geeignete Art und Weise formuliert sein, beispielsweise als Tabletten, Kapseln, Pillen, Granulat oder Pulver zum Auflösen. Dazu werden die Inhaltsstoffe in bekannter Weise, gegebenenfalls unter Verwendung von Hilfsstoffen wie Lactose, Saccharose, Magnesiumsteorat, Talkum zu Tabletten gepreßt. Die Formulierung eines derartigen Präparats erfolgt nach an sich bekannten galenischen Vorschriften und unter Einarbeitung an sich bekannter Excipientien. Bevorzugt wird das erfindungsgemäße Mittel in einer Form, die zur Auflösung in Wasser geeignet ist, formuliert.

Die Kaliumsalze können als Pulver zum Auflösen in Wasser zur Anwendung kommen; auch als Brausetabletten können sie eingesetzt werden. Dabei werden die Citrate bzw. Lactate in situ hergestellt, indem die Brausetablette beispielsweise Kalium- und/oder Natriumhydrogencarbonat und Citronensäure und/oder Milchsäure enthält. Die Kaliumcitrate bzw. -lactate bilden sich nach dem Auflösen der Brausetablette in Wasser durch Reaktion des Hydrogencarbonats mit Citronensäure bzw. Milchsäure. Um den Geschmack zu verbessern, können übliche Geschmacks- und Aromastoffe eingesetzt werden, gegebenenfalls zusammen mit anderen Hilfsstoffen, wie Farbstoffen, Stabilisatoren, Süßstoffen etc.

Die folgenden Beispiele erläutern die Erfindung näher.

### Beispiele

### Beispiel 1

Das erfindungsgemäße Mittel wird hergestellt, indem innig miteinander vermischt werden:
3,25 g Trikaliumcitrat (= 30 mmol Kalium)
4,50 g Calcium-L-lactathydrat (600 mg Calcium)
10 *µ*g Vitamin D₃ (Cholecalciferol)
sowie Aromastoffe und Saccharin.

Das Mittel wird in Wasser (ca. 100 bis 200 ml) gelöst und getrunken.

Eine Gruppe von zehn postmenopausalen Frauen nimmt zehn Tage lang täglich das erfindungsgemäße Mittel ein, anschließend werden von den Frauen zehn Tage lang täglich 4,50 g Calcium-L-lactathydrat (600 mg Calcium) plus 10 *µ*g Vitamin D₃ zum Vergleich eingenommen. In beiden Versuchsperioden wird die Calciumausscheidung im Urin gemessen und damit die Calciumbilanz erstellt.

### Ergebnis:

### Erfindungsgemäßes Gemisch: Mittlere Calciumausscheidung im Urin/Tag: 203 mg Calcium

### Vergleichsgemisch (ohne Kaliumcitrat): Mittlere Calciumausscheidung im Urin/Tag: 265 mg Calcium

Verbesserung der Calciumbilanz um ca. 60 mg/Tag. Dies entspricht ca. 20 g Calcium pro Jahr. Da das menschliche Skelettsystem ca. 1000 g Calcium enthält, wird daher die Abbaurate um ca. 2 % verringert. Zudem stieg während der Einnahme des erfindungsgemäßen Mittels Osteocalcin (im Serum) von 4,8 ng/ml auf 5,9 ng/ml an, während dann Osteocalcin nach Einnahme des Vergleichspräparates wieder auf 5,1 ng/ml abgesunken war.

### Beispiel 2

Der Versuch des Beispiels 1 wird wiederholt, indem nun zwei Gruppen von je zehn postmenopausalen Frauen jeweils ein Jahr mit dem erfindungsgemäßen Gemisch des Beispiels 1 bzw. mit Calciumlactat plus Vitamin D₃ (ohne Kaliumcitrat) behandelt werden. Nach einem Jahr werden die Knochenverluste in % bestimmt.

| | Knochenverlust in % |
|---|---|
| Calcium + Vitamin D₃ | - 1,2 % |
| erfindungsgemäßes Mittel | + 1,5 % |

Während mit der üblichen Behandlung immer noch ein Knochenverlust auftrat, konnte mit dem erfindungsgemäßen Mittel sogar eine Steigerung der Knochenmasse erreicht werden.

### Beispiel 3

Das erfindungsgemäße Mittel wird hergestellt, indem miteinander innig vermischt werden:
3,25 g Trikaliumcitrat (= 30 mmol Kalium)
4,50 g Calcium-L-lactathydrat (600 mg Calcium)
10 *µ*g Vitamin D₃ (Cholecalciferol)
120 *µ*g Vitamin K₁ (Phyllochinon)
sowie Aromastoffe und Saccharin.

Das Mittel wird in Wasser (ca. 100 bis 200 ml) gelöst und getrunken.

Eine Gruppe von zehn postmenopausalen Frauen wird nun jeweils 14 Tage lang behandelt mit:
I) 4,50 g Calcium-L-lactathydrat plus 10 *µ*g Vitamin D₃ (Vergleichsgruppe)
II) Erfindungsgemäßes Mittel nach Beispiel 1
III) Erfindungsgemäßes Mittel nach Beispiel 3

Es werden bestimmt: Calciumausscheidung im Urin, Osteocalcium im Serum, Hydroxyprolinausscheidung im Urin. (Ein höheres Osteocalcin bedeutet einen erhöhten Knochenaufbau, während eine höhere Hydroxyprolinausscheidung auf eine Verstärkung des Knochenabbaus hinweist).

| | Gruppe I | Gruppe II | Gruppe III |
|---|---|---|---|
| Calcium im Urin (pro Tag) | 261 mg | 197 mg | 175 mg |
| Osteocalcin | 5,2 ng/ml | 6,2 ng/ml | 7,3 ng/ml |
| Hydroxyprolin im Urin (pro Tag) | 30 mg | 27 mg | 24,5 mg |

Die Ergebnisse zeigen, daß das erfindungsgemäße Gemisch nach Beispiel 1 (Gruppe II) den Knochenabbau hemmt und die zusätzliche Gabe von Vitamin K₁ (Gruppe III) eine zusätzliche günstige Wirkung im Hinblick auf eine Verminderung des Knochenabbaus hervorruft.

### Beispiel 4

Es werden in üblicher Weise unter Verwendung der Hilfsstoffe Talkum, Magnesiumstereat, Aromen und Saccharin Lutschtabletten gepreßt. Eine Tablette enthält:
1,50 g Trikaliumcitrat
2,00 g Calcium-L-Lactat-Hydrat
3 *µ*g Vitamin D₃ (Cholecalciferol)
60 *µ*g Vitamin K₁ (Phyllochinon)

10 Männer im Alter von mehr als 60 Jahren nehmen über 2 Wochen täglich jeweils 3 Lutschtabletten zu sich (Gruppe I). Nach einer Pause von 2 Wochen nehmen die Männer täglich als Nahrungsergänzung 4,5 g Calcium-L-Lactat-Hydrat plus 9 *µ*g Vitamin D₃ über 2 Wochen (Gruppe II). Unter dem erfindungsgemäßen Gemisch (Gruppe I) betrug die durchschnittliche tägliche Calciumbilanz + 44 mg Calcium/Tag, unter dem Vergleichsgemisch dagegen nur + 5 mg Calcium/Tag.

## Patentansprüche

1. Mittel zur Prophylaxe und/oder Therapie von Zuständen, die mit einer Störung der Knochenbildung einhergehen, enthaltend
i) Kaliumcitrat, -lactat und/oder -malat,
ii) Vitamin D und
iii) ein Calciumsalz.

2. Mittel nach Anspruch 1, dadurch gekennzeichnet, daß es weiterhin Vitamin K enthält.

3. Mittel nach Anspruch 1 oder 2, dadurch gekennzeichnet, daß das Calciumsalz Calcium-L-lactathydrat ist.

4. Mittel nach mindestens einem der Ansprüche 1 bis 3, dadurch gekennzeichnet, daß es 10 bis 100 mmol Kaliumcitrat bzw.-lactat, bezogen auf Kalium, enthält.

5. Mittel nach Anspruch 4, dadurch gekennzeichnet, daß es 5 bis 20 mmol Trikaliumcitrat, entsprechend 15 bis 60 mmol Kalium, enthält.

6. Mittel nach mindestens einem der Ansprüche 1 bis 5 zur Prophylaxe und/oder Therapie des Knochenabbaus.

7. Verwendung der wie in den Ansprüchen 1 bis 5 definierten Zusammensetzung zur Herstellung eines Mittels zur Prophylaxe und/oder Therapie von Zuständen, die mit einer Störung der Knochenbildung einhergehen, sowie zur Herstellung eines Mittels zur Prophylaxe und/oder Therapie des Knochenabbaus.

## Claims

1. Agent for preventing and/or treating conditions associated with a disturbance of bone formation, containing
i) potassium citrate, lactate and/or malate,
ii) vitamin D and
iii) a calcium salt.

2. Agent according to claim 1, characterized in that it also contains vitamin K.

3. Agent according to claim 1 or 2, characterized in that the calcium salt is calcium L-lactate hydrate.

4. Agent according to at least one of claims 1 to 3, characterized in that it contains 10 to 100 mmol of potassium citrate or lactate, based on potassium.

5. Agent according to claim 4, characterized in that it contains 5 to 20 mmol of tripotassium citrate, corresponding to 15 to 60 mmol of potassium.

6. Agent according to at least one of claims 1 to 5 for preventing and/or treating bone demineralization.

7. Use of the composition as defined in claims 1 to 5 for the preparation of an agent for preventing and/or treating conditions associated with a disturbance of bone formation, and for the preparation of an agent for preventing and/or treating bone demineralization.

## Revendications

1. Substance destinée à prophylaxie et/ou à la thérapie d'états inhérents à des troubles d'ossification, contenant
i) du citrate, du lactate et/ou du malate de potassium,
ii) de la vitamine D, et
iii) un sel de calcium.

2. Substance selon la revendication 1, caractérisée en ce que qu'elle contient en outre de la vitamine K.

3. Substance selon la revendication 1 ou 2, caractérisée en ce que le sel de calcium est du L-lactatehydrate de calcium.

4. Substance selon l'une au moins des revendications 1 à 3, caractérisée en ce qu'elle contient 10 à 100 mmoles de citrate ou de lactate de potassium rapportées au potassium.

5. Substance selon la revendication 4, caractérisée en ce qu'elle contient 5 à 20 mmoles de citrate de tripotassium, correspondant à 15 à 60 mmoles de potassium.

6. Substance selon l'une au moins des revendications 1 à 5 destinée à la prophylaxie et/ou à la thérapie de la dégradation osseuse.

7. Utilisation de la composition définie dans les revendications 1 à 5 pour la fabrication d'une substance destinée à la prophylaxie et/ou à la thérapie d'états inhérents à des troubles d'ossification, ainsi que pour la fabrication d'une substance destinée à la prophylaxie et/ou à la thérapie de la dégradation osseuse.
